(19) Europäisches Patentamt / European Patent Office / Office européen des brevets

(11) **EP 3 062 858 B1**

(12) **EUROPEAN PATENT SPECIFICATION**

(45) Date of publication and mention
of the grant of the patent:
**10.10.2018 Bulletin 2018/41**

(21) Application number: **14792810.5**

(22) Date of filing: **01.11.2014**

(51) Int Cl.:
**A61M 16/06** $^{(2006.01)}$

(86) International application number:
**PCT/EP2014/073506**

(87) International publication number:
**WO 2015/063283 (07.05.2015 Gazette 2015/18)**

(54) **SEALING CUSHION FOR A PATIENT INTERFACE**

ABDICHTUNGSPOLSTER FÜR EINE PATIENTENSCHNITTSTELLE

COUSSIN D'ÉTANCHÉITÉ DESTINÉ À UNE INTERFACE PATIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.11.2013 EP 13191279**

(43) Date of publication of application:
**07.09.2016 Bulletin 2016/36**

(73) Proprietor: **Koninklijke Philips N.V.**
**5656 AE Eindhoven (NL)**

(72) Inventors:
- **ASVADI, Sima**
  **NL-5656 AE Eindhoven (NL)**
- **VONCKEN, Rudolf Maria Jozef**
  **NL-5656 AE Eindhoven (NL)**
- **KLEE, Mareike**
  **NL-5656 AE Eindhoven (NL)**
- **HAARTSEN, Jacob Roger**
  **NL-5656 AE Eindhoven (NL)**
- **WILLARD, Nicolaas Petrus**
  **NL-5656 AE Eindhoven (NL)**
- **HENDRIKS, Cornelis Petrus**
  **NL-5656 AE Eindhoven (NL)**
- **ZANTEN, Joyce van**
  **NL-5656 AE Eindhoven (NL)**

(74) Representative: **Schudelaro, Antonius Adrianus Petrus**
**Philips Intellectual Property & Standards**
**High Tech Campus 5**
**5656 AE Eindhoven (NL)**

(56) References cited:
| WO-A1-2012/153289 | WO-A1-2013/001489 |
| US-A1- 2005 199 239 | US-A1- 2006 096 598 |
| US-A1- 2009 223 518 | US-A1- 2011 088 699 |
| US-A1- 2011 146 684 | US-A1- 2012 055 485 |
| US-A1- 2012 138 061 | |

**Description**

FIELD OF THE INVENTION

The present invention relates to a sealing cushion for a patient interface that may be particularly used in a pressure support system for providing a flow of breathable gas to a patient.

BACKGROUND OF THE INVENTION

Patient interfaces, such as facial mask in pressure support systems, are used for delivering a flow of breathable to a user. Such gases like air, cleaned air, oxygen or any modification thereof are submitted to the user (also referred to as patient) via the patient interface in a pressurized or unpressurized way.

For several chronic disorders and diseases the usage of such a patient interface is necessary or at least advisable.

One non-limiting example of such a disease is obstructive sleep apnea or obstructive sleep apnea syndrome (OSA). OSA is usually caused by an obstruction of the upper airway. It is characterized by repetitive pauses in breathing during sleep and is usually associated with a reduction in blood oxygen saturation. These pauses in breathing, called apneas, typically last 20 to 40 seconds or longer. The obstruction of the upper airway is usually caused by reduced muscle tonus of the body that occurs during sleep. The human airway is composed of walls of soft tissue which can collapse and thereby obstruct breathing during sleep. Tongue tissue moves towards the back of the throat during sleep and thereby blocks the air passages. OSA is therefore commonly accompanied with snoring.

Different invasive and non-invasive treatments for OSA are known. One of the most powerful non-invasive treatments is the usage of Continuous Positive Airway Pressure (CPAP) or Bi-Positive Airway Pressure (Bi-PAP) in which a patient interface, e.g. a facial mask, is connected via a hose to a pressure generator that blows pressurized breathable gas into the patient interface and through the airway of the patient in order to keep it open. Usually, a long-term use of the patient interface is necessary. The patient interface is in most of the cases worn during the night while the patient is asleep.

Examples for patient interfaces are:

- nasal masks, which fit over the nose and deliver gas through the nasal passages,
- full face masks, which fit over both, the nose and the mouth, and deliver gas to both, and
- nasal pillows, which are regarded as masks as well within the scope of the present invention and which comprise small nasal inserts that deliver the gas directly to the nasal passages.

In order to guarantee a reliable operation, the patient interface needs to closely fit on the patient's face to provide an air-tight seal at the mask-to-face interface. The patient interface is donned to the patient's head by means of a headgear with straps that go around the back of the patient's head. The patient interface usually comprises a soft sealing cushion that is used as mask-to-patient interface, i.e. that contacts the face of patient when the patient interface is worn. Furthermore, such patient interfaces usually comprise a rigid or semi-rigid holding structure for holding the sealing cushion in place and for supplying mechanical stability to the patient interface. This holding structure is denoted as mask shell. Still further, such patient interfaces may comprise one or more forehead supports with additional cushions. These forehead supports are configured to contact the forehead of the patient in order to balance the forces with which the patient interface is pressed against the patient's face.

One of the most important parts of such a patient interface is the above-mentioned sealing cushion. This sealing cushion has the function to form an air-tight seal between the patient interface and the patient's face that is needed for a good sealing effect. Apart from the basic functionality the air-tight contact is also needed to prevent excessive leakage as it can be an additional source of patient discomfort. Since the sealing cushion is in direct contact with the patient's face, the properties of said sealing cushion also have a major influence on the overall comfort of the patient interface for the patient.

An implication of an incorrectly fitted sealing cushion is the formation of red marks in the patient's face. Such red marks can stay visible after the mask has been removed for a time period ranging from several minutes to many hours. Repeated use of the mask can cause even more sever skin damage similar to pressure ulcers.

The severity of the red marks is also a function of skin properties and subcutaneous tissue structure which may vary for different facial locations. Boney parts of the face are the most vulnerable parts to red mark formation. Especially on the nose bridge pressure peaks are most often observed due to the nose geometry and certain skin characteristics, such as the thickness of the stratum corneum (the upper most layer of the skin) in combination with the bone structure of the nose. Therefore, the highest contact stresses between the sealing cushion and the patient's face are usually observed in the area of the nose bridge (apex of the nose).

Some known prior art documents proposed to use very soft cushion parts in the nose bridge contacting area. US 2012/285464 A1 proposes a sealing cushion, wherein the area of the sealing cushion that contacts the nose bridge during use is arranged to be more flexible than the remaining parts of the cushion. Even though these measures showed an improvement, experiments have shown that such sealing cushions still result in a formation of red marks, especially when the patient interface is used over longer time periods.

WO 2013/001489 A1 discloses a cushion member for a user interface device. The cushion member is structured to provide a load distribution functionality responsive to the cushion member being donned by the user, wherein at least a portion of the cushion member has a local stiffness of less than or equal to 100 kPa/mm responsive to a stress increase on the cushion member of 1 kPa - 15 kPa.

Further sealing cushions which are designed to be as soft as possible are known from US 2012/0138061 A1 and US 2006/0096598 A1.

Experiments of the applicant have shown that providing softer or more flexible materials only partly solves the above-mentioned problem, but does not completely prevent a red mark formation on the patient's nose bridge. Thus, there is still room for improvement.

SUMMARY OF THE INVENTION

It is an object of the present invention to provide a patient interface and a cushion for such a patient interface that reduces the formation of pressure points and red marks on the face of the patient.

According to the present invention this object is solved by a sealing cushion for a patient interface, wherein the cushion comprises a nose bridge contacting area that is configured to contact a nose bridge of a patient during use of the cushion, wherein the nose bridge contacting area comprises a material having a secant stiffness of less than 0.2 N/mm at a material strain ranging between 0.1 and 1, wherein the secant stiffness is defined as tensile force applied to the material per unit width of the material divided by the material strain, wherein the material strain denotes a ratio of material extension in length per unit of original length of the material.

According to a further aspect of the present invention, the object is solved by a patient interface comprising a sealing cushion of the above-mentioned type and a support member for holding the sealing cushion.

According to a still further aspect of the present invention, the object is solved by a pressure support system comprising a patient interface with a cushion of the above-mentioned type and a pressure generator connected to the patient interface for delivering a flow of breathable gas via the patient interface to the airway of a patient.

Preferred embodiments of the invention are defined in the dependent claims. It shall be understood that the claimed patient interface and the claimed pressure support system have similar and/or identical preferred embodiments as the claimed sealing cushion and as defined in the dependent claims.

In view of the above-mentioned object most of the known prior art documents like US 2012/285464 A1 propose to provide a sealing cushion with a nose bridge contacting area having a material that is softer, more flexible or more viscoelastic than the material used for the remaining parts of the sealing cushion. However, the inventors of the present invention have found that parameters like softness and viscoelasticity are not the most important ones for preventing a formation of red marks or pressure points. Experiments of the applicant have shown that the most important parameter for the material used for the nose bridge contacting area is the stiffness of the material, in particular the secant stiffness of the material. The herein defined secant stiffness denotes the tensile force per unit width of the material needed for a certain level of material extension/stretching. The experiments of the applicant have particularly shown that a formation of red marks and pressure points on the patient's face may be prevented if a material is used for the nose bridge contacting area of the sealing cushion that has a secant stiffness of less than 0.2 N/mm at a material strain ranging between 0.1 and 1. Materials which meet these secant stiffness requirements do not result in a formation of red marks or pressure points on the nose bridge of the patient at all even after a long-term use of the patient interface.

It shall be noted that materials which meet the above-mentioned secant stiffness range are less stiff and therefore more stretchable than materials that are "usually" used for sealing cushions of the type mentioned above. The function of this lower stiffness/higher stretchability in the nose bridge area of the cushion is two-fold: First, it facilitates a good sealing effect for different nose dimensions and geometries, since such materials conform better to the three-dimensional shape of the nose and hence prevent leakage. Secondly, these materials prevent a creation of high contact stresses for nose geometries that do no perfectly fit to the design and shape of the cushion in the nose bridge area, since the material easily stretches to conform to the nose size and shape. A reduction and/or elimination of high contact stress points consequently leads to a reduction and/or elimination of red marks on the nose bridge of the patient.

A low secant stiffness value means a that the material extension is quite high at a given tensile force per unit width, or that a comparatively low tensile force per unit width of the material is needed to be applied for a certain level of material extension/stretching. A material with low secant stiffness is thus more stretchable than a material with a higher secant stiffness.

As mentioned above, the secant stiffness is a type of tensile material stiffness that is measured as tensile force applied to the material per unit width of the material divided by the material strain:

$$S_S = \frac{F}{w\varepsilon}$$

wherein $S_S$ is the secant stiffness, F the force applied to the material, w the width of the material and $\varepsilon$ the material strain. The material strain $\varepsilon$ herein denotes the ratio of change in length of the material per unit of original length in unstrained condition of the material. Thus, a strain of zero means that the material is in unstrained condition and has its original length, whereas a strain of 1 or 100% means that the material is stretched to such an extent that the received length is twice as large as the original unstrained length of the material.

Furthermore, it shall be noted that the secant stiffness is measured per unit width of the material, in contrast to Young's modulus which is usually measured as stress divided by strain, i.e. as force divided by strain per unit cross-sectional area of the material (not per unit width of the material as measured herein). Within the meaning of the present invention the width of the material denotes the dimension of the material perpendicular to the length and the thickness.

Still further, it is important to note that within the meaning of the present invention "at least" the part of the cushion that contacts the nose bridge of the patient (herein denoted as nose bridge contacting area) should comprises the above-mentioned type of material. The present invention is, however, not limited to a cushion that comprises the above-mentioned type of material only in the nose bridge contacting area. According to the present invention other parts of the cushion or even the whole cushion may be made of the above-mentioned type of material.

The secant stiffness as defined above is thus influenced by both the material properties, such as e.g. Shore hardness, and by the geometry, such as the thickness of the material and its shape into which it is formed to conform with the nose bridge area of the patient's face.

The formation of pressure points and red marks in the very sensitive nose bridge area of the patient's face can still be more effectively prevented if materials are used in the nose bridge contacting area of the sealing cushion that have an even lower secant stiffness than defined above, i.e. that are even more stretchable.

Even though WO 2013/001489 A1 also uses the term "stiffness", the therein defined "stiffness" relates to a different material parameter than the secant stiffness defined according to the present disclosure. The "stiffness" defined in WO 2013/001489 A1 is, in contrast to the "secant stiffness", not defined per unit width of the material, but per unit of cross-sectional area. Even more important is that the stiffness defined in WO 2013/001489 A1 relates to a material behavior that is indicative of a compressibility of the material (reduction of thickness during loading), whereas the secant stiffness defined according to the present disclosure relates to a material behavior that is indicative of the stretchability (material extension in length during loading). In other words, the stiffness defined in WO 2013/001489 A1 much more relates to the softness and compressibility of the material instead of to the stretchability of the material.

US 2012/0138061 A1 is also silent about a material for a sealing cushion having a secant stiffness as defined according to the present disclusore. US 2012/0138061 A1 makes use of a cushion that is molded in a material having a Type A durometer of about 35 to about 45. This material parameter again refers to the softness, but not to the stretchability, i.e. not to the secant stiffness of the material as defined according to the present disclosure. Apart from that, US 2012/0138061 A1 tries to regulate the softness and stiffness of the sealing cushion by means of a dual-wall configuration with an undercushion and a covering membrane (i.e. not by adapting the material behavior of the cushion). Still further, the term "stiff" used in US 2012/0138061 A1 relates to a different kind of material behavior that may not be compared to the secant stiffness defined in the present disclosure. The term "stiffness" in US 2012/0138061 A1 denotes the flexibility behavior of the material, i.e. how bendable the material is.

US 2006/0096598 A1 discloses a sealing cushion comprising a thermo-plastic elastomer gel having certain defined material properties like: Hardness, Elongation, Young's Modulus and Tear Strength. None of these material parameters may be compared to the secant stiffness defined according to the present disclosure. "Hardness" refers to the shore hardness, i.e. how hard or soft a material is. "Elongation" is according to US 2006/0096598 A1 defined as change of length relative to the original length before the material starts breaking. "Young's modulus" and "Tear Strength" is also not the same as secant stiffness. Young's modulus is defined as tensile force applied to the material per unit area (i.e. not per unit width of the material). In contrast to Young's modulus, the secant stiffness is thus influenced by the thickness of the material and its shape. Tear Strength is a parameter that is measured in a standard test and indicative of a force that is necessary to initiate a tear within the material.

According to embodiments of the present invention, the material of the nose bridge contacting area has a secant stiffness of less than 0.1 N/mm, and most preferably of less than 0.05 N/mm at a material strain between 0.1 and 1. Again, the afore-mentioned material is not restricted to be used only in the nose bridge contacting area, but may also be comprised in other parts of the cushion. The comfort of the patient may even be increased if the more than the nose bridge contacting area of the cushion comprises the afore-mentioned material.

The term "at a material strain between 0.1 and 1" means that the secant stiffness is below the mentioned upper limit in the range of material strain between 0.1 and 1, preferably but limited over the whole range of material strain between 0.1 and 1. However, it should be noted that such materials do not necessarily have to have a linear secant stiffness behavior in said strain range.

According to a further embodiment, the sealing cushion comprises a receiving opening for receiving a part of the patient's face, wherein the sealing cushion comprises at least two sections surrounding the receiving opening, a first section building the nose bridge contacting area and a second section that is configured to contact parts of the patient's face other than the nose bridge. In this case said second section is designed to have a higher/larger secant stiffness than the first section.

In case of a full face mask the first section contacts the nose bridge of the patient, whereas the second section of the sealing cushion contacts the remaining areas around the nose and the mouth of the patient. In case of a nose mask the first section contacts the nose bridge of the patient, while the second section of the sealing cushion surrounds the remaining parts around the nose of the patient. Even though it would be generally conceivable to use a material with the above-mentioned tensile stiffness properties for the entire cushion, i.e. not only for the nose bridge contacting area, it is advantageous to use a different kind of materials that is less stretchable and has a higher secant stiffness for the remaining parts of the sealing cushion that do not contact the nose bridge of the patient. Using stiffer/less stretchable materials in the latter mentioned areas especially has the advantage that the overall stability of the sealing cushion is increased. An increased stability also helps to prevent leakage in the remaining areas, so that the important sealing behavior of the sealing cushion may be accomplished.

The first and the second section are preferably seamlessly connected with each other. Such a seamless connection also helps to prevent leakage at the boundary between the two sections having different secant stiffness/stretchability properties. A seamless connection furthermore increases the comfort for the patient, as the patient might not even recognize that two different materials are used along the circumference of the sealing cushion. It should be also noted that the sealing cushion may comprise more than two sections with materials of different tensile stiffness properties.

According to a further embodiment, the nose bridge contacting area is formed as a sealing flap. This means that the above-mentioned material forming the nose bridge contacting area may be realized as a sealing flap that, for example, covers an insert provided within the interior of the sealing cushion. Said insert may comprise a soft material, e.g. made of a gel or any other viscoelastic material. In this case the sealing flap forms the outer surface of the sealing cushion that contacts the nose bridge of the patient.

A material that may be used for the sealing flap in the nose bridge contacting area may comprise silicone. Experiments of the applicant have shown that a silicone material having a Shore A value of less than 40 and a thickness of less than 0.5 mm (in unstrained condition) meets the above-mentioned requirement of having a secant stiffness value of less than 0.45 N/mm in the strain range between 10% and 100%. Again it shall be noted that the above-mentioned secant stiffness that shall be met does not only depend on the Shore A hardness but also on the thickness of the material. It shall be furthermore noted that the above-mentioned combination of Shore A hardness and material thickness leads to an inherently softer and more stretchable material than the standard silicone materials that are used for sealing cushions according to the prior art. Experiments of the applicant have shown that silicone materials with a Shore A value of 5 and a thickness of less than or equal to 0.5 mm are even more suitable for preventing red marks on the nose bridge of the patient.

However, a formation of red marks on the nose bridge of the patient may also be prevented if other materials are used for the nose bridge contacting area of the sealing cushion, as long as they meet the above-mentioned secant stiffness requirements. It has been shown that these requirements may also be met if the material of the nose bridge contacting area comprises a stretchable textile material, such as a knitted or woven fabric. Alternatively, the material can be made out of stretchable nonwoven material or any other form of fibre assembly. The tensile stiffness/secant stiffness of such fabrics may even be decreased if they contain a stretchable or elastic yarn, such as spandex or elastane. Additionally, if the stretchable nose bridge material has the desired secant stiffness as defined previously in the text in both warp and weft directions for a woven material and wale and course directions for a knotted material it will offer enhanced comfort and lower chance of red marks formation

In a still further embodiment, the nose bridge contacting area comprises a knitted or woven fabric (with or without elastic yarn) that is coated with a polymeric material such as silicone material, preferably with a silicone material having a Shore A value of smaller or equal to 5 and a thickness preferably of smaller or equal to 0.1 mm. Such textile materials that are coated with thin layers of silicone or any other stretchable polymers further ensure a high air-tightness and leakage prevention in the nose bridge area.

BRIEF DESCRIPTION OF THE DRAWINGS

These and other aspects of the invention will be apparent from and elucidated with reference to the embodiment(s) described hereinafter. In the following drawings

Fig. 1 schematically illustrates an embodiment of a pressure support system for providing a pressurized flow of breathable gas to the airway of a subject;
Fig. 2 shows an embodiment of a patient interface that may be used in the pressure support system shown in Fig. 1;
Fig. 3 shows the patient interface according to the embodiment shown in Fig. 2 from the side (Fig. 3a) and from below (Fig. 3b) in order to show an embodiment of a sealing cushion according to the present invention in more detail;
Fig. 4 shows experimental results of tested materials regarding their stretchability behavior; and
Fig. 5 shows desired stretchability zones of materials that are preferably used for a nose bridge contacting area of the sealing cushion.

DETAILED DESCRIPTION OF THE INVENTION

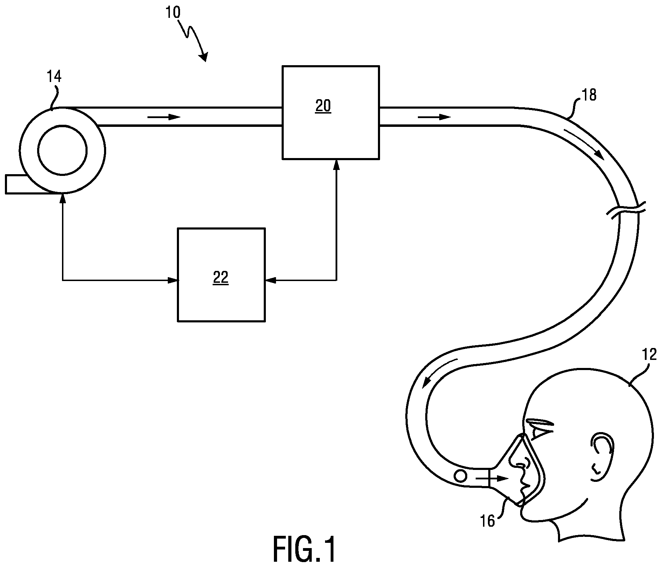

Fig. 1 illustrates an embodiment of a pressure support system according to an embodiment. The pressure support system is in Fig. 1 in its entirety denoted with reference numeral 10. The system 10 is configured to deliver a pressurized flow of breathable gas to the airway of a subject 12 (herein also denoted as patient 12). The pressurized flow of breathable gas may be provided in accordance with a certain therapy regimen that is designed to treat a respiratory disease, such as Obesity Hyperventilation Syndrome (OHS), Obstructive Sleep Apnea (OSA), and/or other respiratory diseases. The therapy regimen calls for maintenance of an average tidal volume, maintenance of a respiratory rate, and/or maintenance of a positive inspiratory and expiratory pressure.

The system 10 is configured to provide therapy to the patient 12 to ensure that the patient 12 breathes at a therapeutic respiratory rate. Examples of such treatments are the usage of Continuous Positive Airway Pressure (CPAP) or Bi-Positive Airway Pressure (BiPAP), in which the pressurized flow of breathable gas is either supplied in a continuous manner (in CPAP machines) or at a high level pressure when the patient inhales and a low level pressure when the patient exhales (in BiPAP machines). The system 10 may also be configured such that spontaneous breaths may be supported at a pressure that is lower than a pressure for breaths that are not spontaneous and are triggered automatically based on the therapeutic respiratory rate.

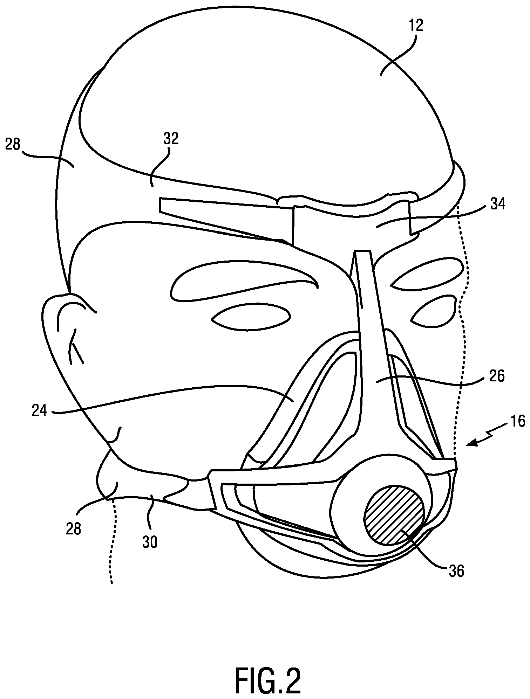

The pressurized flow of breathable gas is generated by a pressure generator 14. The pressurized flow of breathable gas is supplied to the patient 12 via a patient interface 16 that is connected to the pressure generator 14 via a hose 18. The patient interface 16 usually has a form of a mask as it will be illustrated in more detail below with reference to Fig. 2. Furthermore, one or more sensors 20 may be provided that measure one or more parameters of the pressurized flow of breathable gas within the hose 18 and/or close to the position of the connection of the patient interface 16 to the hose 18. These flow parameters may be transferred to a processing unit 22 that is configured to control the pressure generator 14. In this way the pressure generator 14 may control parameters of the pressurized flow of breathable gas, for example the flow, the pressure, the volume, the humidity, the temperature and/or the gas composition, depending on the one or more parameters measured with the one or more sensors 20. The parameters measured by the one or more sensors 20 may e.g. include a measured temperature, pressure, flow rate and/or gas composition within the hose 18.

Fig. 2 shows an embodiment of the patient interface 16. In this certain embodiment the patient interface 16 is realized as a full face mask that during use covers the mouth and nose of the patient 12. However, it shall be noted that patient interfaces 16 within the scope of the present invention may also be realized as any other mask type, such as e.g. a nasal mask.

The patient interface 16 comprises a cushion 24. The main function of the cushion 24 is the provision of a mask-to-face interface as it contacts the face of the patient 12 when the patient interface 16 is worn by the patient 12. Within this function the cushion 24 also has to provide an air-tight sealing, so that in the best case the pressurized flow of breathable gas is only delivered to the respiratory passages (nose and/or mouth) of the patient 12 without leaking through gaps in between the cushion 24 and the face of the patient 12. The cushion 24 is therefore herein also denoted as sealing cushion 24. It should be clear that in order to provide the above-mentioned sealing effect, it is necessary that the shape of the sealing cushion 24 conforms in an optimal way with the facial contours of the patient 12.

As can be further observed from Fig. 2, the sealing cushion 24 is connected to and held by a support member 26. This support structure 26, which is often also denoted as mask shell 26, serves as a holder for the sealing cushion 24 in order to provide stability to the patient interface 16. The mask shell 26 preferably also comprises a connector 36 to which the hose 18 is connected. However, the hose 18 may also be directly connected to the sealing cushion 24.

In order to attach the patient interface 16 to the patient's head, the patient interface 16 usually further comprises a headgear 28. In the embodiment shown in Fig. 2 the headgear 28 comprises two straps 30, 32 with which the patient interface 16 may be donned to the patient's face. The length of these headgear straps 30, 32 is preferably adjustable, such that the force with which the sealing cushion 24 is pressed against the face of the patient 12 may be controlled. This force has to be usually quite high in order to prevent an unwanted leakage that may cause a malfunction of the patient interface 16. On the other hand, these high forces may lead to a considerably high pressure with which the patient interface 16, in particular the sealing cushion 24, is pressed to the patient's face.

Since such patient interfaces 16 are usually worn on a long-term basis (during the whole night) this may often cause pressure points that are visible in the form of red marks on the face of the patient. These red marks are not only unattractive but may also hurt and can even lead to pressure ulcers or other tissue damages. The most sensitive area is the area on and around the nose bridge of the patient 12, since the skin is in this area rather thin and bony parts occur directly below the skin. Of course, red marks and pressure sores can also be created on other areas of the face e.g. at the check or under the nose.

In order to create more stability of the mask, the patient interface 16 may furthermore comprise a forehead support 34. The present invention mainly focuses on preventing a formation of red marks on the nose bridge of the patient 12 by providing a special type of sealing cushion 24, as this will be explained in the following.

It is to be noted that the present invention is not restricted to the mask type that is exemplarily shown in Fig. 2. The present invention may also be implemented in a nasal mask, for example.

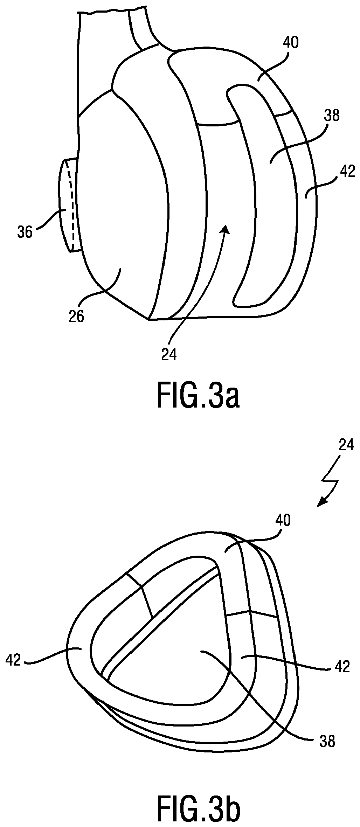

Figs. 3a and 3b show the sealing cushion 24 according to the present invention in two different schematical views. The sealing cushion 24 defines a receiving opening 38 for receiving a part of the patient's face. In the illustrated case this receiving opening 38 is configured to receive the nose and the mouth of the patient 12. In case of a nasal mask the receiving opening 38 would only be configured to receive the nose of the patient 12.

An important aspect of the present invention is that the sealing cushion 24 is divided into separate sections. These separate sections may consist of different materials in order to provide a different tensile stiffness and stretchability behavior over the different sections along the parameter of the sealing cushion 24.

A first section or part 40 forms the so-called nose bridge contacting area that is configured to contact the nose bridge (the apex of the nose) of the patient 12 during use of the patient interface 16. This first section 40 is arranged in upper part of the sealing cushion 24. The second section 42 may form the remaining parts of the sealing cushions, i.e. the parts that do not contact the nose bridge of the patient 12 during use.

Since the most contact pressure occurs at and around the nose bridge of the patient 12 it might be evident to use a softer material for the first section 40 than for the second section 42. However, the inventors of the present invention have found that the softness of the material is not the most important parameter. It is much more important how stretchable the material is in said first section 40, which depends on the tensile stiffness of the material. It has been found that said first section 40 needs to comprise a low secant stiffness in order to prevent too high contact pressures and to prevent a formation of red marks that may result therefrom. Materials with a low secant stiffness are highly stretchable and allow decreasing the high contact stresses as they may conform almost perfectly to the facial contours around the nose bridge due to their stretching behavior. On the other hand, such highly stretchable materials facilitate a good sealing effect for different nose dimensions and geometries through having better draping and conforming to the three-dimensional shape of the nose. Hence, leakage may also be prevented in this area.

The rest of the sealing cushion 24, i.e. the second section 42 does not need to be as stretchable as the first section 40 of the sealing cushion 24, i.e. the material may have a higher tensile stiffness in the second section 42 than in the first section 40. Materials with a higher tensile stiffness (secant stiffness) may be used in the second section 42, since the tissue in the remaining facial regions is less vulnerable and a material of higher tensile stiffness may in these regions provide an improved sealing effect.

In order to increase the patient comfort it is preferred that the two sections 40, 42 of the sealing cushion 24 are seamlessly connected with each other. Both sections 40, 42 may be realized as flaps that consist of different materials. According to another alternative, the second section 42 may be made of the same material as the first section 40. In this case the cushion 24 does not even need to have separate sections 40, 42, but may be, for example, fully made of the material explained below. However, it shall be noted that the sealing cushion 24 may comprise also more than two different sections 40, 42, as long as the first section 40 that builds the nose bridge contacting area has the following properties:

Experiments of the applicant have shown that a formation of pressure points and red marks on the nose bridge of the patient 12 may be prevented, as long as a material is used for the nose bridge contacting area 40 that has a secant stiffness of less than 0.45 N/mm. Said secant stiffness denotes the level of tensile force that needs to be applied to the material per unit with of the material in order to reach a certain amount of material strain. It is measured as the fraction of tensile force per unit width of the material divided by the material strain. The material strain denotes a ration of change in length per unit of original length of the material. The secant stiffness as defined herein is thus comparable with Young's modulus with the difference that Young's modulus is defined as stress over strain, i.e. as tensile force applied to the material divided by material strain per unit cross sectional area of the material (not per unit with of the material).

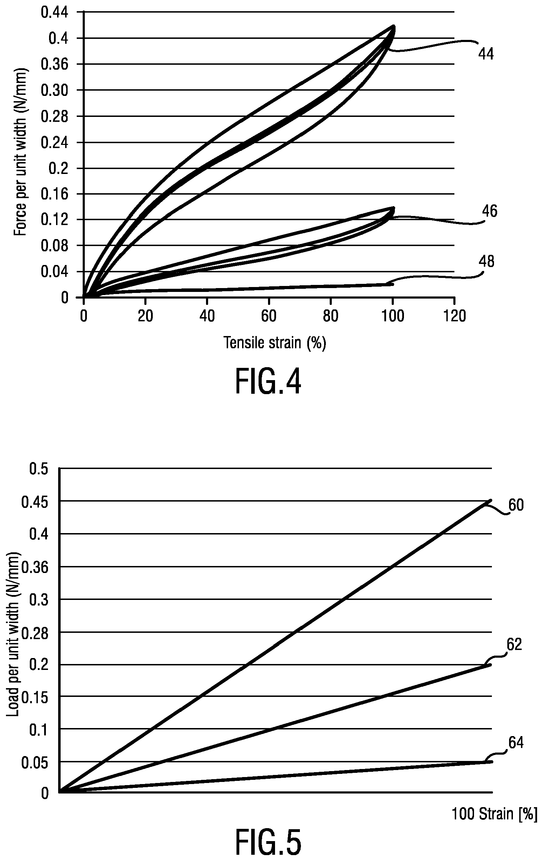

The above-mentioned upper limit of having a secant stiffness of 0.45 N/mm has been found after testing different types of materials that were used for the nose bridge contacting area 40 of the sealing cushion 24. The result of a tensile hysteresis testing of selected polymeric materials and selected textile materials, both as uncoated textiles and after coating them with a silicone layer at various Shore values and thicknesses, is shown in the graph illustrated in Fig. 4.

Fig. 4 demonstrates some graphs of strain (in %) versus tensile load (force) per unit width of several materials. Reference numeral 44 depicts a material that has been tested for the nose bridge contacting area 40 of the sealing cushion 24 in a long-term study. Tested material 44 was a silicone material with a Shore A value of 40 and had a thickness of 0.5 mm. The long-term study performed by the applicant showed that such kind material was not stretchable enough, at least not over the whole range of strain values between 1% and 100% strain. When using such kind of material some red marks still occurred on the nose bridge of the patient 12 after a long-term use of the patient interface 16.

Other tested materials, which are in the graph of Fig. 4 denoted by reference numerals 46 and 48, did not result in a red mark formation on the nose bridge even when they were used over longer time periods. Reference numeral 48 depicts a silicone material having a Shore A value of 5 and a thickness of 0.2 mm. As it can be seen from the graph such a material has a secant

# 

stiffness (see at 100% strain) of around 0.02 N/mm. The secant stiffness may be seen in the graph as quotient of the y-value over the x-value. The term "secant stiffness" is derived from the fact that its value may be seen as the slope of a secant between the origin of the graph and the point on the curve under consideration. In contrast thereto, the "tangent stiffness" would be defined as the slope of the tangent on the curve shown in Fig. 4. The secant stiffness, which is according to the present invention an important factor, only equals the tangent stiffness for a linear stiffness curve. This is in practice, however, rather unusual.

Reference numeral 46 depicts several types of fabrics that have been coated with a thin silicone coating having a Shore A value of 5 and a thickness of 90 μm. As it can be seen from the graph in Fig. 4, these materials have a secant stiffness (see at 100% strain) of around 0.1 N/mm.

Based on these results the following may thus be concluded: The determining characteristic of the material that would help to reduce red marks on and around the nose bridge of the patient 12 is its ability to reduce contact stress points on the nose bridge. This is governed by the secant stiffness of the material that is defined as its behavior in tensile deformation. The inventors of the present invention have identified that if materials are used for the nose bridge contacting area 40 that have a secant stiffness of less than 0.45 N/mm at a material strain between 0.1 and 1, i.e. between 10% and 100%, a formation of red marks can be significantly reduced or even completely prevented.

The above-mentioned preferred zone regarding the secant stiffness is schematically illustrated by graph 60 in Fig. 5. Even more preferable would be a material with a secant stiffness of less than 0.2 N/mm at a material strain between 10% and 100%. This zone is indicated in Fig. 5 with reference numeral 62. Most preferable would be a material with a secant stiffness of less than 0.05 N/mm at a material strain between 10% and 100%. The latter mentioned stretchability zone is indicated in Fig. 5 by reference numeral 64.

It shall be noted that the zone are in Fig. 5 indicated as linear zones. The secant stiffness behavior of the material, however, does not have to be linear in the indicated strain range between 10% and 100%, as long as the stiffness curve is within one of the indicated zones 60, 62, 64. It is to be noted that material 44 does not fall into the indicated zone 60 over the whole strain range between 10% and 100% strain. In consequence, this means that if silicone is used, the silicone material has to have a Shore A value of less than 40 and a thickness of less than 0.5 mm in order to meet the requirement of having a secant stiffness of less than 0.45 N/mm over the strain range of 10 to 100%, and in order to prevent a red mark formation on the nose bridge. Other materials that have been found to meet the above-mentioned secant stiffness requirements are knitted, woven or nonwoven fabrics, preferably with a silicone coating thereon.

Furthermore, it has been shown to be advantageous if said fabrics comprise an elastic yarn, such as spandex or elastane.

**Claims**

1. A sealing cushion (24) for a patient interface (16), wherein the cushion (24) comprises a nose bridge contacting area (40) that is configured to contact a nose bridge of a patient (12) during use of the cushion (24), wherein the nose bridge contacting area (40) comprises a material having a secant stiffness of less than 0.2 N/mm at a material strain between 0.1 and 1, wherein the secant stiffness is defined as tensile force applied to the material per unit width of the material divided by the material strain, wherein the material strain denotes a ratio of material extension in length per unit of original length of the material.

2. The sealing cushion according to claim 1, wherein the material of the nose bridge contacting area (40) has a secant stiffness of less than 0.05 N/mm at a material strain ranging between 0.1 and 1.

3. The sealing cushion according to claim 1, wherein the sealing cushion (24) comprises a receiving opening (38) for receiving a part of the patient's face, wherein the sealing cushion (24) comprises at least two sections (40, 42) surrounding the receiving opening (38), a first section building the nose bridge contacting area (40) and a second section (42) that is configured to contact parts of the patient's face other than the nose bridge, wherein said second section (42) has a higher secant stiffness than the first section (40).

4. The sealing cushion according to claim 3, wherein the first and the second section (40, 42) are seamlessly connected with each other.

5. The sealing cushion according to claim 1, wherein the nose bridge contacting area (40) is formed as a sealing flap.

6. The sealing cushion according to claim 1, wherein the material of the nose bridge contacting area (40) comprises a polymeric material, preferably a silicone material.

7. The sealing cushion according to claim 6, wherein the polymeric material has a Shore A value of less than 40 and a thickness of less than 0.5 mm.

8. The sealing cushion according to claim 1, wherein the material of the nose bridge contacting area (40) comprises a fabric.

9. The sealing cushion according to claim 8, wherein the fabric is a knitted or woven fabric.

10. The sealing cushion according to claims 6 and 8, wherein the fabric is coated with the polymeric material, preferably with a silicone material.

11. The sealing cushion according to claim 8, wherein the fabric comprises an elastic yarn.

12. The sealing cushion according to claim 11, wherein the elastic yarn comprises spandex or elastane.

13. A patient interface (16) comprising a sealing cushion (24) as claimed in claim 1 and a support member (26) for holding the sealing cushion (24).

14. A pressure support system (10) comprising a patient interface (16) as claimed in claim 13 and a pressure generator (14) connected to the patient interface (16) for delivering a flow of breathable gas via the patient interface (16) to the airway of a patient (12).

**Patentansprüche**

1. Abdichtungspolster (24) für eine Patientenschnittstelle (16), wobei das Polster (24) einen Nasenrückenkontaktbereich (40) umfasst, der dazu konfiguriert ist, einen Nasenrücken eines Patienten (12) während eines Gebrauchs des Polsters (24) zu kontaktieren, wobei der Nasenrückenkontaktbereich (40) ein Material umfasst, dass eine Sekantensteifigkeit von weniger als 0,2 N/mm bei einer Materialdehnung zwischen 0,1 und 1 aufweist, wobei die Sekantensteifigkeit definiert ist als eine auf das Material pro Einheitsbreite des Materials aufgebrachte Zugkraft dividiert durch die Materialdehnung, wobei die Materialdehnung ein Verhältnis einer Materiallängenzunahme pro Einheit einer ursprünglichen Länge des Materials bezeichnet.

2. Abdichtungspolster nach Anspruch 1, wobei das Material des Nasenrückenkontaktbereichs (40) eine Sekantensteifigkeit von weniger als 0,05 N/mm bei einer Materialdehnung in einem Bereich zwischen 0,1 und 1 aufweist.

3. Abdichtungspolster nach Anspruch 1, wobei das Abdichtungspolster (24) eine Aufnahmeöffnung (38) zur Aufnahme eines Teils des Gesichtes des Patienten umfasst, wobei das Abdichtungspolster (24) wenigstens zwei Abschnitte (40, 42) umfasst, welche die Aufnahmeöffnung (38) umgeben, einen ersten Abschnitt, der den Nasenrückenkontaktbereich (40) bildet, und einen zweiten Abschnitt (42), der dazu konfiguriert ist, andere Teile des Patientengesichts als den Nasenrücken zu kontaktieren, wobei der zweite Abschnitt (42) eine höhere Sekantensteifigkeit aufweist als der erste Abschnitt (40).

4. Abdichtungspolster nach Anspruch 3, wobei der erste und der zweite Abschnitt (40, 42) nahtlos miteinander verbunden sind.

5. Abdichtungspolster nach Anspruch 1, wobei der Nasenrückenkontaktbereich (40) als eine Abdichtungsklappe ausgebildet ist.

6. Abdichtungspolster nach Anspruch 1, wobei das Material des Nasenrückenkontaktbereichs (40) ein Polymermaterial umfasst, vorzugsweise ein Silikonmaterial.

7. Abdichtungspolster nach Anspruch 6, wobei das Polymermaterial einen Shore A-Wert von weniger als 40 und eine Dicke von weniger als 0,5 mm aufweist.

8. Abdichtungspolster nach Anspruch 1, wobei das Material des Nasenrückenkontaktbereichs (40) ein Gewebe umfasst.

9. Abdichtungspolster nach Anspruch 8, wobei das Gewebe ein gewirktes oder ein gewebtes Gewebe ist.

10. Abdichtungspolster nach den Ansprüchen 6 und 8, wobei das Gewebe mit dem Polymermaterial beschichtet ist, vorzugsweise mit einem Silikonmaterial.

11. Abdichtungspolster nach Anspruch 8, wobei das Gewebe ein elastisches Garn umfasst.

12. Abdichtungspolster nach Anspruch 11, wobei das elastische Garn Spandex oder Elastan umfasst.

13. Patientenschnittstelle (16) mit einem Abdichtungspolster (24) nach Anspruch 1 und einem Stützelement (26) zum Halten des Abdichtungspolsters (24).

14. Druckunterstützungssystem (10), umfassend eine Patientenschnittstelle (16) nach Anspruch 13 und einen mit der Patientenschnittstelle (16) verbundenen Druckerzeuger (14) zur Abgabe eines Atemgasstroms über die Patientenschnittstelle (16) an den Atemweg eines Patienten (12).

**Revendications**

1. Coussin d'étanchéité (24) destiné à une interface patient (16), dans lequel le coussin (24) comprend une zone de contact avec l'arête du nez (40) qui est configurée pour entrer en contact avec une arête du nez d'un patient (12) pendant une utilisation du coussin (24), dans lequel la zone de contact avec l'arête du nez (40) comprend un matériau ayant une rigidité sécante inférieure à 0,2 N/mm pour une contrainte de matériau entre 0,1 et 1, dans lequel la rigidité sécante est définie comme une force de traction appliquée au matériau par largeur unitaire du matériau divisée par la contrainte de matériau, dans lequel la contrainte de matériau désigne un rapport d'extension de matériau en longueur par unité de longueur initiale du matériau.

2. Coussin d'étanchéité selon la revendication 1, dans lequel le matériau de la zone de contact avec l'arête du nez (40) a une rigidité sécante inférieure à 0,05 N/mm pour une contrainte de matériau se situant entre 0,1 et 1.

3. Coussin d'étanchéité selon la revendication 1, dans lequel le coussin d'étanchéité (24) comprend une ouverture réceptrice (38) pour recevoir une partie du visage du patient, dans lequel le coussin d'étanchéité (24) comprend au moins deux sections (40, 42) entourant l'ouverture réceptrice (38), une première section constituant la zone de contact avec l'arête du nez (40) et une seconde section (42) qui est configurée pour mettre en contact des parties du visage du patient autres que l'arête du nez, dans lequel ladite seconde section (42) a une rigidité sécante supérieure à celle de la première section (40).

4. Coussin d'étanchéité selon la revendication 3, dans lequel la première et la seconde section (40, 42) sont reliées uniformément l'une à l'autre.

5. Coussin d'étanchéité selon la revendication 1, dans lequel la zone de contact avec l'arête du nez (40) est formée comme un rabat d'étanchéité.

6. Coussin d'étanchéité selon la revendication 1, dans lequel le matériau de la zone de contact avec l'arête du nez (40) comprend un matériau polymère, de préférence un matériau en silicone.

7. Coussin d'étanchéité selon la revendication 6, dans lequel le matériau polymère a une valeur Shore A inférieure à 40 et une épaisseur inférieure à 0,5 mm.

8. Coussin d'étanchéité selon la revendication 1, dans lequel le matériau de la zone de contact avec l'arête du nez (40) comprend un tissu.

9. Coussin d'étanchéité selon la revendication 8, dans lequel le tissu est un tissu tricoté ou tissé.

10. Coussin d'étanchéité selon les revendications 6 et 8, dans lequel le tissu est revêtu avec le matériau polymère, de préférence avec un matériau en silicone.

11. Coussin d'étanchéité selon la revendication 8, dans lequel le tissu comprend un fil élastique.

12. Coussin d'étanchéité selon la revendication 11, dans lequel le fil élastique comprend du spandex ou de l'élasthanne.

13. Interface patient (16) comprenant un coussin d'étanchéité (24) selon la revendication 1 et un élément de support (26) pour maintenir le coussin d'étanchéité (24).

14. Système de support à pression (10) comprenant une interface patient (16) selon la revendication 13 et un générateur de pression (14) relié à l'interface patient (16) pour distribuer un flux de gaz respirable via l'interface patient (16) dans les voies respiratoires d'un patient (12).

FIG.1

FIG.2

FIG.3a

FIG.3b

FIG.4

FIG.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012285464 A1
- WO 2013001489 A1
- US 20120138061 A1
- US 20060096598 A1